# EUROPEAN PATENT APPLICATION

(11) **EP 1 358 878 A1**
(43) Date of publication of application: **05.11.2003**
(21) Application number: 02715747.8
(22) Date of filing: 16.01.2002
(51) Int. Cl.: A61K 31/198, A61K 38/02, A61P 29/00, A61P 19/02

(54) **REMEDIES/PREVENTIVES FOR INFLAMMATORY DISEASES**

(30) Priority: 30.01.2001 JP 2001021643
(71) Applicant: Ajinomoto Co., Inc., Tokyo 104-8315 (JP)
(72) Inventor: YONEDA, Junya Cent. Res. Lab. Ajinomoto Co. Inc., Kawasaki-shi, Kanagawa 210-0801 (JP); MURATA, Yukie Cent. Res. Lab. Ajinomoto Co. Inc., Kawasaki-shi, Kanagawa 210-0801 (JP); HAMURO, Junji Cent. Res. Lab. Ajinomoto Co. Inc., Kawasaki-shi, Kanagawa 210-0801 (JP)
(74) Representative: Nash, David Allan
(86) International application number: JP0200224
(87) International publication number: WO02060431

(57) **Abstract**

Excellent preventives/remedies for inflammatory diseases are provided by using ornithine and/or branched amino acid(s) as an active ingredient. Moreover, a method of treating or preventing inflammatory diseases by using these agents and utilization (use) of the active ingredients in preventives/remedies for inflammatory diseases are provided. As the amino acid(s) (ornithine and branched amino acid (s)) to be used as the active ingredient, use can be made of not only free amino acid (s) but also salt (s) thereof, and derivative (s) thereof which can be converted into the amino acids serving as the active ingredient (free form) in vivo, if any. Combined use of ornithine with branched amino acid(s) can establish a particularly enhanced effect. These ingredients, which have no or little side effect, are usable in the form of medicines (including transfusions, supplements, etc.), or foods and/or drinks (including medical foods, health foods, specified health foods, etc.), particularly for treating or preventing diseases such as rheumatoid arthritis, and for preventing the progress of these diseases, ameliorating the same, etc.

## Description

### TECHNICAL FIELD

The present invention relates to novel remedies/preventives for inflammatory diseases, precisely to agents (drugs) (compositions) that contain ornithine and/or branched amino acid(s) (in any form of free amino acid(s), salt(s) thereof and derivative (s) thereof convertible into free amino acid(s) *in vivo*) as an active ingredient and are useful for treating and/or preventing inflammatory diseases such as typically arthritic diseases, rheumatic diseases, especially chronic rheumatism (rheumatoid arthritis), and for preventing the progress of these diseases and/or ameliorating them, and/or the like (hereinafter referred to as "remedy(ies)/preventive(s) for inflammatory disease(s)" in the present invention). The invention can provide medicines (including transfusions, supplements (nutrient preparations), etc.) or foods or drinks (which are foods and/or drinks)(including medical foods, health foods, specific (specified) health foods, etc.) which are safe (having no or little side effect) and have such excellent effects. Further, the invention relates to a method of treating inflammatory diseases (including treating and ameliorating inflammatory diseases, and preventing the progress of these diseases, etc.), or preventing them, and to a use of the specific active ingredients for remedies/preventives for inflammatory diseases (including treatment and amelioration of inflammatory diseases and prevention of the progress of the diseases).

### BACKGROUND ART

Rheumatic diseases are the generic term of diseases that cause pains at joints and around them. There are a large number of rheumatic diseases, which may be classified, on the basis of the cause thereof, into immunological disorders (chronic rheumatism, systemic lupus erythematosus, etc.), infections (bacterial arthritis, etc.), allergies (serum sickness, etc.), biochemical and endocrine disorders (gout, hyperparathyroidism, etc.), retrograde changes (osteoarthritis, spondylitis deformans), traumatic and neurotic diseases (traumatic arthritis, diabetes, shoulder and arm syndrome, etc.), hereditary and congenital diseases (congenital hip dysplasia, etc.), tumors (multiple myeloma, etc.), and others (amyloidosis, etc.). Their pathological models include synovitis (chronic rheumatism), cartilage deformation (osteoarthritis), crystallization-inducing arthritis (gout), arthritic infections (bacterial arthritis), enthesopathy (ankylosing spondylitis), tendonitis (calcifying tendonitis), myositis (dermatomyositis), and psychosomatic factors (fibrositis).

The prevalence of rheumatic diseases increases more in more aging societies, and it is at least 10 % in advanced countries. In particular, the prevalence of osteoarthritis increases in persons of 20 years or older with the increase in their age, and about a half or more of 50-year-old persons (50 to 59 years old persons) may suffer from the disease including slight injuries. One essential symptom of rheumatic diseases in clinical observations includes pains at joints and therearound, but depending on the type thereof, the diseases may produce various complication symptoms in systemic organs. For treating rheumatic diseases, the principle is that the causal disorder to cause the arthritic symptoms is identified and the diseases are treated in consideration of the symptoms. The symptomatic treatment for arthritic symptoms includes oral or parenteral administration of a non-steroid anti-inflammatory medicine or a steroid medicine to retard the inflammation and the pain, combined with physical treatment of cooling or warming the affected part, etc. A case with functional disorders may undergo kinesitherapy or may be fitted with a medical tool, or may undergo surgical therapy with an artificial joint. In case where his/her joint disorder lasts long, the patient may suffer from mental disorder, and may require an antidepressant or specific therapy by specialists such as psychological counselors, etc.

The current medical therapy in the art is described in detail hereinunder.

For treating rheumatic diseases (hereinafter referred to as "RA"), first selected are non-steroid anti-inflammatory drugs (NSAIDs). These days, however, the role of NSAIDs for treatment of RA is being reduced. This is because NSAIDs are expected to be analgesic for RA but are not antirheumatic. In addition, another reason is because the side effects of NSAIDs such as typical digestive tract injury, renal hypofunction and others are not negligible clinically. In that situation, disease-modifying antirheumatic drugs (DMARDs) such as auranofin, penicillamine and others are used in early stages. The recent tendency in the art is toward use of new antirheumatic drugs such as methotrexalate (MTX), FK506 and others. These drugs act on the immune system to correct the abnormality thereof, but most of their effects and functions are unclear as yet and must be clarified in future. Owing to the serious side effects thereof, DMARDs have not been used so much in early stages, but these days they are often used by medical specialists in very early stages.

With the rapid progress these days in analyzing the causes and the symptoms of RA, novel treatment methods are being developed, including anticytokine therapy, oral peptide therapy, antisense therapy, anti-adhesion molecule antibody therapy, etc. On the other hand, however, no one knows at present medicines, foods and drinks for prevention of chronic rheumatism. Heretofore, remedies for relieving the symptoms of developed RA have been essentially developed, but no one knows preventives capable of administering to patients who do not develop symptoms of RA as yet. It is now desired to develop remedies/preventives essentially for preventing local inflammations caused by RA. For such cases that have a high possibility of genetically presenting RA symptoms and the like, it is desired to develop foods and drinks capable of being orally taken for preventing the diseases.

### PROBLEM TO BE SOLVED BY THE INVENTION AND OBJECT OF THE INVENTION

For treating rheumatic diseases, the principle is that the causal disorder to cause the arthritic symptoms is identified and the diseases are treated in consideration of the symptoms. However, there are many different types of causal disorders of the diseases, and most of them are basically intratable disorders and it is often difficult to treat them. Accordingly, the basic treatment for the diseases at present is how to relieve the pain caused by the diseases by the use of drugs for symptomatic treatment such as non-steroid anti-inflammatory medicines or steroid medicines or through physical treatment. However, most of rheumatic diseases are intractable ones and take a long period of time for their treatment. Therefore, the drug treatment must be with those of little side effects that are usable for a long period of time, but the non-steroid anti-inflammatory medicines and steroid medicines that are used at present have various side effects and their long-term use has many problems. For example, the non-steroid anti-inflammatory medicines may cause serious gastric hemorrhage, peptic ulcer, renal disorders, etc. The steroid medicines may have various many side effects of worsen infections, peptic ulcer, osteoporosis and others, owing to its immunosupression action. The physical treatment is merely to temporarily relieve the pain caused by the diseases.

Given that situation as above, a problem to be solved by the present invention or an object of the present invention is to develop excellent remedies/preventives for inflammatory diseases having the effects as above, which are highly safe and can be orally administered or orally taken as foods or drinks (foods and/or drinks). In particular, the invention is to develop agents (drugs) which are favorable also for foods or drinks and of which the essential object is to prevent local inflammations caused by RA mentioned above.

### DISCLOSURE OF THE INVENTION

We, the present inventors have found that a composition that contains ornithine or a branched amino acid (leucine, isoleucine, valine, etc.) is highly effective in oral administration to typical animal models with arthritis, and have further found that agents (drugs) that contain such an amino acid as an active ingredient are extremely effective for treating and preventing inflammatory diseases, especially arthritic diseases and rheumatic diseases, and for preventing the progress of such diseases and ameliorating them, and have few side effects, and are therefore favorable for medicines (including transfusions, supplements (nutrient preparations), etc.) and for foods or drinks (foods and/or drinks) (including medicinal foods, health foods, specified health foods, etc.). On the basis of these findings, we have completed the present invention.

Specifically, the present invention resides in remedies/preventives (which are remedies and/or preventives) for inflammatory diseases (agents (drugs) for treating and/or preventing inflammatory diseases, and also for preventing the progress of the diseases and/or for ameliorating them), which contain at least any one of ornithine and branched amino acid (s) as the active ingredient.

The ornithine and the branched amino acid(s) each may be in any form of free amino acid(s), salt(s) thereof and derivative (s) thereof, if any, that can be converted into free amino acid(s) *in vivo.*

In case where the amino acids are in the form of salts for use herein, the salts acceptable for foods and/or drinks or for medicines may be selected from the salts of the amino acids for the active ingredient.

The ornithine and the branched amino acid(s) that serve as the active ingredient include optical isomer(s), and the type of the optical isomer(s) thereof are not specifically defined for use in the invention. Preferred are L-isomer(s), as they occur in nature.

The branched amino acid(s) include leucine, isoleucine, valine, etc. At least one (one or more) of ornithine, leucine, isoleucine and valine is preferably employed for the active ingredient for use in the invention.

Preferably, the active ingredient is a mixed amino acid that comprises ornithine and at least one (one or more) branched amino acid of leucine, isoleucine and valine. More preferably, it comprises all these four amino acids.

The diseases to which the agents (drugs) of the invention are applied are inflammatory diseases. Typically, they include arthritic diseases and rheumatic diseases.

In the invention, amino acid derivatives (ornithine derivatives, leucine derivatives, isoleucine derivatives, valine derivatives, etc.) capable of being converted into the corresponding amino acids *in vivo* can be used as the ornithine or the branched amino acid(s) that serve as the active ingredient. For their examples, herein mentioned are peptides that contain the active ingredient amino acid(s) as the constitutive amino acid(s) in their molecules preferably as many as possible.

The products of the invention may be used parenterally, but especially when orally administered or taken, they well exhibit the above-mentioned excellent effects. Therefore, they are favorable for oral administration or oral taking. Accordingly, they can be readily used in the form of medicines or foods or drinks (foods and/or drinks).

The medicines may be in any form including ordinary oral pharmaceutical preparations, parenteral pharmaceutical preparations, as well as other preparations such as supplements (nutrient preparations), transfusions, etc. On the other hand, the foods and/or drinks may also be in any form including ordinary foods and drinks that are expected or requested to exhibit the above-mentioned effects, as well as medical foods, health foods, specified health foods, etc.

The agents (drugs) of the invention mentioned above may contain any other active ingredient (s) (of the same type (s) or different type (s)) and additive(s) so long as they contain the above-mentioned active ingredient in the present invention and exhibit the intended effect(s) and activity (ies) also mentioned above, and these are within the scope of the invention.

Another aspect of the present invention resides in a method of treating or preventing inflammatory diseases, which comprises administering ornithine and/or branched amino acid(s) to a living body or making the same taken by a living body.

(The ornithine and the branched amino acid(s). each may be in any form of free amino acid(s), salt(s) thereof and derivative(s) thereof, if any, that can be converted into free amino acid(s) *in vivo.*)

For the embodiment (form) of administering the amino acid(s) to a living subject (body) or making them (the amino acid(s)) taken by a living subject, employable are the above-mentioned remedies/preventives (remedies and/or preventives) for inflammatory diseases. Especially preferably, they are administered to or taken by the body in the form of their medicines (supplements, transfusions, etc.) or foods or drinks (medical foods, health foods, specified health foods, etc.). The method is favorable for prevention or treatment of arthritic diseases.

Still another feature (aspect) of the present invention resides in a use of ornithine and/or branched amino acid(s) (active ingredient) for (in) remedies/preventives (remedies and/or preventives) for inflammatory diseases, preferably remedies/preventives for arthritic diseases, and for their productions.

(The ornithine and the branched amino acid(s) each may be in any form of free amino acid(s), salt(s) thereof and derivative(s) thereof, if any, that can be converted into free amino acid(s) *in vivo.*)

The remedies/preventives for inflammatory diseases for the use are as in the above description. As so mentioned hereinabove, one preferred embodiment of their use is in the form of medicines (supplements (nutrient preparations), transfusions, etc.) or foods and/or drinks (medical foods, health foods, specified health foods, etc.), or in the form wherein the remedies/preventives are used in medicines, or foods and/or drinks.

### BRIEF DESCRIPTION OF THE DRAWINGS

### Fig. 1:

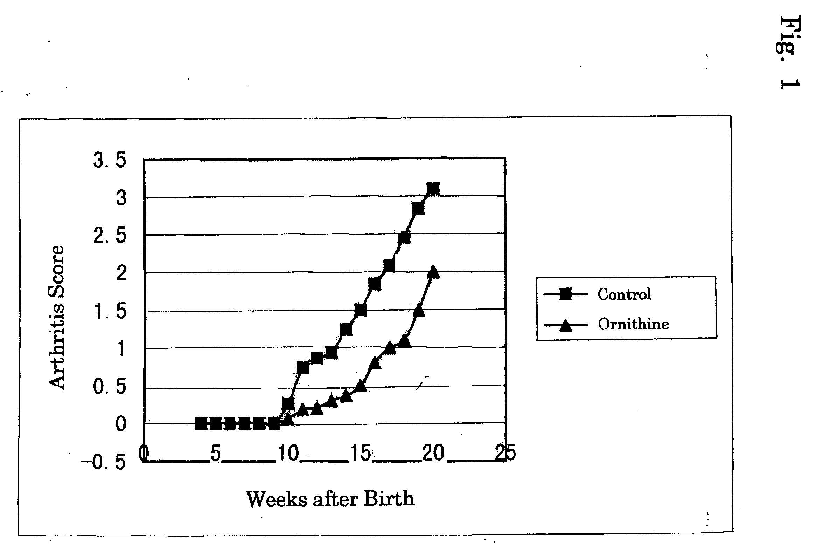

Fig. 1 is a graph showing the effect of ornithine administration to SKG mice with spontaneously developed chronic rheumatism obtained in Example 3, in which L-ornithine (free form) was administered to the SKG mice and mice were scored for arthritis on days indicated, as described.

Regarding the score, 0 indicates no change; 0.1 indicates reddish edema seen in the finger joints of the mice; 0.5 indicates edema seen in the joints of the four limbs thereof; and 1.0 indicates serious edema seen in the joints thereof. For these, the mice were visually checked. The total of the scores of all the four limbs of each mouse is plotted in the graph.
■: control, ▲: ornithine.

### Fig. 2:

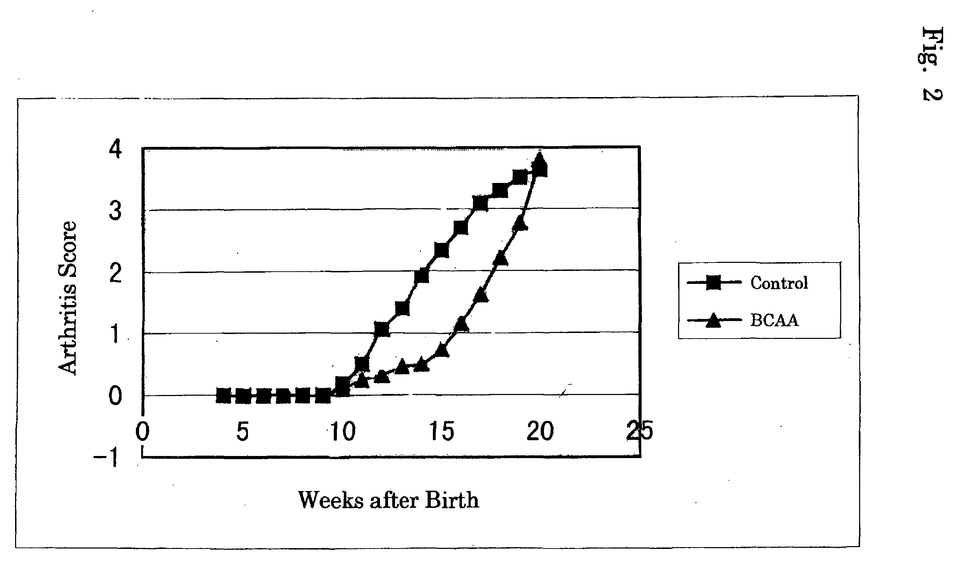

Fig. 2 is a graph showing the effect of administration of a branched-chain amino acid (branched amino acid) mixture BCAA to SKG mice with spontaneously developed chronic rheumatism obtained in Example 4, in which BCAA was administered to the SKG mice and mice were scored for arthritis on days indicated, as described.

Regarding the score, 0 indicates no change; 0.1 indicates reddish edema seen in the finger joints of the mice; 0.5 indicates edema seen in the joints of the four limbs thereof; and 1.0 indicates serious edema seen in the joints thereof. For these, the mice were visually checked. The total of the scores of all the four limbs of each mouse is plotted in the graph.
■: control, ▲: BCAA.

### Fig. 3:

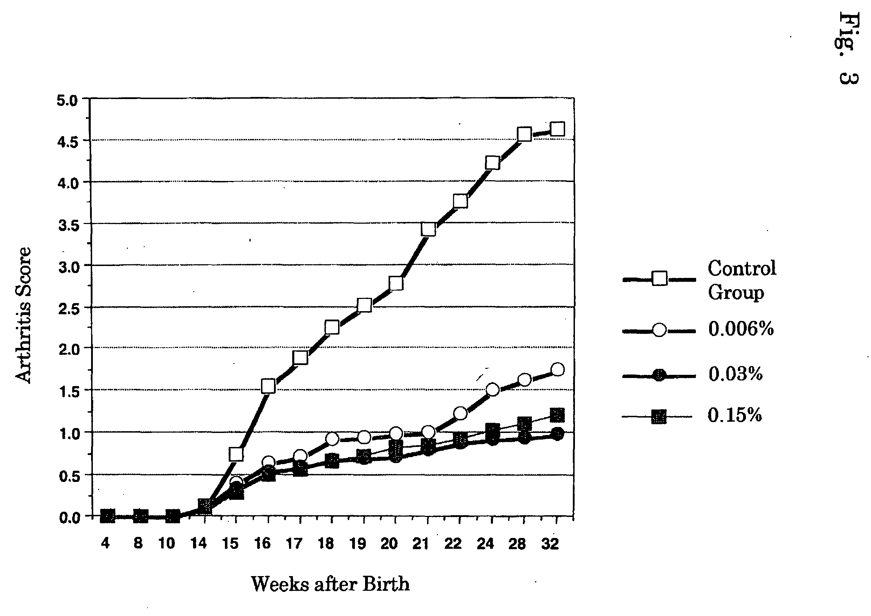

Fig. 3 is a graph showing the effect of retarding chronic rheumatism in SKG mice obtained in Example 6, in which the concentration of ornithine administered to the mice was varied. L-ornithine (free form) was orally administered to SKG mice, and mice were scored for arthritis on days indicated, as described. Three different doses of L-ornithine were tried, and the dose dependence of the ornithine effect was checked.

Regarding the score, 0 indicates no change; 0.1 indicates reddish edema seen in the finger joints of the mice; 0.5 indicates edema seen in the joints of the four limbs thereof; and 1.0 indicates serious edema seen in the joints thereof. For these, the mice were visually checked. The total of the scores of all the four limbs of each mouse is plotted in the graph. □: control group, ○: 0.006 %, ●: 0.03 %, and ■: 0.15 % (The numerals all indicate the ornithine concentration (% by weight) in the aqueous solution administered to the mice.)

### MODES FOR CARRYING OUT THE INVENTION

Modes for carrying out the invention are described hereinunder.

Ornithine (L-form) is one type of basic amino acids in protein. Some pharmaceutical and physiological effects of ornithine and branched amino acids such as those mentioned below are known, but nothing is known about them relative to rheumatic diseases.

Ornithine results from amino acid metabolism, and it is a constitutive component of an urea cycle in which it converts ammonia, a central neurotoxin into urea in the liver for detoxication. Ornithine varies its concentration in bodies along with the concentration variation in N-acetylglutamic acid, an allosteric activator for a carbamoyl phosphate synthetase, in accordance with the rapid change in the nitrogen amount therein while eating. Further, ornithine is a starting material for polyamines such as putrescine, spermidine, spermine, etc. Those polyamines are basic and strongly bond to DNA to control its replication, and therefore they have some influences on protein synthesis and cell division. They are much in tissues where protein and nucleic acid synthesis occurs much.

Branched amino acids are hydrophobic amino acids having a branched alkyl side chain, such as leucine, isoleucine, valine, etc. The side chain participates in hydrophobic bonding, and in formation of an active site (binding site) in the pocket of the active center of enzyme, etc. Further, branched amino acids account for 40 % of essential amino acids, and their metabolism has a significant clinical meaning. In those with cirrhosis (hepatic cirrhosis), it is believed that not only the intake of branched amino acids will lower but also the transfer (incorporation) of branched amino acids from blood into flesh (muscular tissue) will be promoted for detoxication of ammonemia (hyperammonemia). Accordingly, in oral replacement therapy with branched amino acids, albumin and the like that the liver synthesizes increase and ameliorates hepatoencephalosis and cirrhosis. In the Examples given hereinunder, the branched amino acids used are in the form of a mixture of three amino acids, L-leucine, L-isoleucine and L-valine, and in particular, these are mixed in a specific ratio of approximately 2:1:1.2 in terms of the ratio of their free amino acids. However, a varying range is accepted for the blend ratio. In case where branched amino acids are used as the active ingredient in the invention, one branched amino acid may be used alone or multiple (two or more) branched amino acids may be used as combined. Naturally, the invention shall cover any mode of using such active ingredients, or such branched amino acids in any desired manner. The branched amino acids may be not only in the form of their free bodies but also in any other form of their salts, if any, or such salts may be combined with free bodies. The salts in such embodiments, which are accepted for medicines or for foods and drinks and which are generally used by those skilled in the art, are all within the scope of the amino acids that are used as the active ingredient in the invention, as one embodiment of the invention.

Of rheumatic diseases, the number of cases with chronic rheumatism (rheumatoid arthritis) is the largest, and the chronic rheumatism is polyarthritis that repeats recrudescence and remission, broadly covering from ambulatory cases to serious cases that are accompanied by joint fracture or extra-articular disorders. Though not as yet clarified, two reasons are now taken into consideration for the disease. One is to result from immune response disorder in lymphocytes and others to the autoantigen existing in joints, especially in synovial membrane or cartilage; and the other is to result from too much promotion of the growth activity of synovial cells themselves for some reason. The essential lesion by RA is in synovial tissues. Concretely, RA results in abnormal growth of synovial membrane to form granulation tissue (pannus), and cartilage and bone are thereby eroded and fractured. In addition, giant cumulative nests of T cells are in the inflamed synovial membrane, and with further development of the inflammation, invasive nests of B cells come to appear. The pathological symptoms of rheumatism are related to T cells and other many cytokines such as IL-1, tumor necrosis factor (TNF) -α and the like that are derived through inflammations. In addition, it is believed that a rheumatic factor known as an antibody to the self-immunoglobulin (Ig)G frequently admitted in cases with rheumatism (rheumatoid patients) may also participate in the pathological symptoms of rheumatism. Further, active oxygen or lysosome enzyme (lysosomal enzyme) is released from the cells of monocytes or macrophages of cases with rheumatism, and it is believed that such active oxygen or lysosome enzyme will also participate in the pathological symptoms such as inflammations seen in rheumatism.

For investigating the method of preventing and treating rheumatic diseases, of which the reason is not as yet clarified, animal models with pathologic symptoms are indispensable. For the animal models with chronic rheumatism, known are models derived through antigen/adjuvant administered, naturally-occurring models, and bioengineering models of mice with gene manipulation.

It is said that laboratory animal models with RA must satisfy at least the following five of histological findings seen in rheumatic synovitis. (a) Growth and hypertrophy of surface layer cells of synovial membrane; (b) infiltration into monocytes, lymphocytes and plasma cells in synovial tissue, and formation of lymphatic follicles therein; (c) fibrin retention; (d) formation of pannus; and (e) erosion and fibrillation of cartilage. In addition to these, other factors are (f) tendency toward chronic synovitis; (g) multiple arthritis; (h) the ratio of females to males with the disease is approximately 3/1, like in RA, or that is, the disease is seen more in females; and (i) familial factor, etc. However, there are a few models that satisfy (f) to (i). Accordingly, in fact, the models that are suitable to the intended studies are selected.

Animal models are generally grouped into two, naturally-occurring models and induced (derived) models. The naturally-occurring models are advantageous in that they develop the intended disease with no artificial operation such as immunization, but many of these are defective in that the disease-developing animals are old-aged and a lot of time is taken for obtaining them and the disease-developing time in them could not be controlled, and the like. On the other hand, the induced models are artificially prepared through sensitization with an antigen or the like, and their advantage is that a desired number of animals can be induced to have the intended disease at any desired time. Popular models that are generally employed in the art are mouse or rat collagen-induced arthritis (CIA) and rat adjuvant arthritis (AA). CIA is chronic multiple arthritis induced in mice or rats through intracutaneous sensitization with the same or different types of II-collagen (IIc: rich in cartilage) along with adjuvant. It is known that BB/DR rats, which have a sequence of high analogy to the RA-sensitive sequence (RASS) commonly admitted in HLA DR1 and DR4 that are said to have a high correlation to human RA, in the polymorphic region of the Dβ chain of RTI of MHC class II, and H-2q or H-2r haplo-type mice (DBA-1, etc.) are readily induced to have the disease. After immunized only once, rats may develop the disease in 10 to 15 days. However, mice generally require boosting (additional) immunization, and from 80 to 100 % of the thus-immunized mice may develop the disease in 10 days to 2 weeks after that. The disease symptoms of the animals last for a few months, and then the animals may be spontaneously cured except that the joint tetanization (ankylosis) and the finger lateral (outside) curvature are still left as they are. AA is multiple arthritis that is induced in rats through intracutaneous sensitization with an oil emulsion of heated died cells of acid-fast bacteria such as tubercle bacilli, and it is widely developed or employed for screening and developing anti-inflammatory medicines and anti-rheumatic medicines. Arthritis exhibits its symptom in approximately 10 to 14 days after the sensitization, as red edema in the joints of the four limbs, and, after having lasted for a few months, its inflammations may be naturally cured with joint tetanization (ankylosis) and deformation being left as such. From the histological viewpoint, AA gives an image of chronic proliferating synovitis and forms pannus, well similar to RA, but it further shows reactive bone growth. Regarding the symptom developing mechanism of AA, the following three opinions are known.
(a) AA is caused by delayed hypersensitive reaction to the cell wall component of bacteria such as peptide glycan (PG); (b) the adjuvant action of PG, especially that of muramyl dipeptide (MDP) on some tissue of the animal gives an autoantigen, and AA is therefore an autoimmune disease; and (c) AA is caused by the cross-reaction resulting from the common antigenicity between the link protein of the proteoglycan of joint cartilage, and bacterial components (especially, thermal (heat) shock protein HSP65).

An SKG mouse model is known as the naturally-occurring model. This is found in BALB/c mouse colonies as a mouse that develops clinical arthritis, and it is sustained through sibship mating. Regarding the thus-mated SKG mice, when they are judged at 6-month age, almost all mice have some clinical arthritis (joint edema) in any of four limb joints. The arthritis is not by vertical/horizontal infection with microorganisms but is by autosomal recessive inheritance mutation. The clinical arthritis (joint edema) starts in around 2 months after the birth essentially at the finger joints of the forepaws, and thereafter extends to the joints of the hands and the joints of the legs. After 6 months, most of the mice develop arthrosclerosis (articular rigidity). From the pathohistological viewpoint, the disease starts from synovitis to periarthritis and further toward pannus formation and cartilage/subcartilaginous bone tissue fracture and fibrillation. This model shows chronic synovitis, and is characterized in that its arthritis does not spontaneously cure, different from the above-mentioned other models. This is an extremely interesting model for studying the mechanism of delayed inflammations. As an engineering model, there is known a transgenic (tg) mouse with a Tax gene of a human T cell leukemia virus-1 (HTLV-1) inserted thereinto. The tg mouse has the transgene expressing in the local joints, in which inflammatory cytokines such as IL-1α, IL-1β IL-6, TNF-α, TGF-β, IFN-γ and IL-2, as well as MHC gene are activated. The mice of the type have an extremely higher immunoreactivity with anti-nuclear antibody, RF, heat shock protein and IIc, than non-tg mice. The tg mouse is a significant model in studies where the influence of Tax gene that will be probably because of its transactivation on the production of inflammatory cytokines or the influence of viruses on the mechanism of autoimmune reaction, and the like are specifically investigated.

It is obvious that T cells play a significant role in RA inflammations, since the RA-inflamed synovial membrane is highly accompanied by T cell invasion, and since the HLA-DR molecule having a specific peptide sequence has a close correlation to the RA symptom development, and the like. T cells that have been activated by some antigen stimulation inside or outside joints produce IL-2, and act to proliferate themselves or other T cells that express an IL-2 receptor CD25. The T cells activated outside the joints temporarily express an early-stage activation factor such as CD25 or CD69, and then, while further expressing VLA-1, they reach vascular endothelial cells in the synovial membrane that strongly express ICAM-1, and then go out of the blood vessels, or that is, inside the synovial membrane to re-express CD69. There is known a polyamine, a factor that retard the production of IL-2 by the activated T cells. The polyamine is a generic term of a linear aliphatic hydrocarbon having at least two primary amino groups, typically including putrescine, spermidine, spermine, etc. The polyamine inhibits the production of IL-2 via hydrogen peroxide, one product to be formed through oxidation with polyamine oxidase. Monocytes produce the polyamine, and retard the IL-2 production by the activated T cells and even the growth of the cells. Ornithine is converted into putrescine by the action of ornithine decarboxylase. Putrescine is converted into spermine or spermidine by the action of spermine synthase or spermidine synthase. The concentration level of spermine or spermidine in the serum of RA cases is higher than that of ordinary persons. Therefore, it is believed that there may act some force of retarding the growth of activated T cells *in vivo.* In the invention, we, the inventors have clarified the possibility that ornithine, a starting material of polyamines, could be a pharmaceutical active ingredient for treating and preventing arthritis by the use of arthritic animal models for three different mechanisms.

On the other hand, "Livact" (Ajinomoto's registered trade mark) that contains L-leucine, L-isoleucine and L-valine (all free amino acids) and provided in the field of medicine as a drug of branched amino acids is used for the cases with uncompensated cirrhosis (decompensated liver cirrhosis) that develop hypoalbuminemia, for ameliorating their hypoalbuminemia. The branched amino acid drug (preparation) retarded clinical arthritis (joint edema) in any of all the three different types of arthritis animal models used in the invention.

The preventives/remedies for inflammatory diseases in the invention are especially useful for treating and/or preventing rheumatic diseases and/or their complications, preventing the progress of these diseases, ameliorating the condition of the diseases, and/or the like. The drugs may contain one or more of the above-mentioned active ingredients either singly or as combined. If desired, the drugs may be administered to patients along with any other remedy for rheumatic diseases that differs from the drugs of the invention in point of the action and the mechanism. The administration route of the drugs of the invention is not specifically defined, and the drugs may be administered in any form, for example, parenterally such as through injection or orally. As acceptable oral administration, the drugs of the invention are advantageous.

The ornithine that is used as the active ingredient in the invention may be in any form of a free body, an ornithine salt, or an ornithine derivative capable of being converted into free ornithine *in vivo.* Similarly thereto, the branched amino acids may also be in any form of free bodies, or, if any, salts of the amino acids, or derivatives of the amino acids capable of being converted into free amino acids *in vivo.* Such ornithine may be combined with at least one such branched amino acid. They may be used in the form of a composition that contains at least the above-mentioned active ingredient. Concretely, when they are administered or given to persons as medicines or foods or drinks, they exhibit their effect of treating or preventing inflammatory diseases, or preventing the progress of the diseases or ameliorating the diseases, or the like, and they are especially effective for treating or preventing arthritic diseases or rheumatic diseases, or preventing the progress of the diseases or ameliorating the diseases, or the like.

The amino acids that serve as the active ingredient in the invention include various optical isomers and their mixtures, which, however, are not specifically defined in the invention in point of their types, etc. Namely, any of optical isomers or racemates are usable in the invention, but L-forms are preferably used as existing in nature.

Ornithine that is used as the active ingredient in the invention may be any and every one that is obtained through hydrolysis of natural protein derived from animals or vegetables, or obtained through fermentation or chemical synthesis. As so mentioned hereinabove, ornithine includes optical isomers of D-form, L-form, DL-form, etc. For use in the invention, preferred is L-form that is one component of protein *in vivo.* Ornithine may be used as it is (free body) or in the form of various salts thereof. The ornithine salt is essentially with an acid, as it is basic. The acid to form salts with ornithine may be any of inorganic acids or organic acids. Examples of the inorganic acids are sulfuric acid, nitric acid, phosphoric acid, hydrohalogenic acid (hydrogen halide aqueous solution) (hydrochloric acid, hydrobromic acid, hydroiodic acid, etc.). Examples of the organic acids are formic acid, acetic acid, propionic acid, oxalic acid, succinic acid, maleic acid, fumaric acid, citric acid, glutamic acid, aspartic acid, gamma-linolenic acid, succinic tocopherol monoester, tocopherol phosphate, ascorbic acid, ascorbylphosphoric acid (phosphate), tocopherol ascorbylphosphate, thioctic acid, N-acetylcysteine, N,N'-diacetylcysteine, lipoic acid, etc. Of their optical isomers, preferred are L-forms.

Ornithine that is used as the active ingredient in the invention includes its derivatives capable of being converted into free ornithine *in vivo.* The derivatives may be any ones which, when taken as foods, drinks or the like containing any of them, can be immediately converted into ornithine (free body) *in vivo.* One example of such derivatives is a peptide (ornithine-peptide) that contains ornithine as its constitutive component. The peptide may have approximately from 2 to 50 amino acids. Preferably, however, the ornithine content of the peptide to be in the drugs for the invention is as high as possible since the intake of peptide dose is in terms of the active ornithine itself. For example, the peptide of the type for use in the invention preferably has an ornithine content, as the constitutive amino acid in the peptide, of at least approximately 10 % (by weight), more preferably at least approximately 30 % (by weight). For the constitutive component of the peptide, ornithine is indispensable as it is the active ingredient itself, but the other amino acids except it are not specifically defined. Preferably, however, the peptide contains many branched amino acids. The peptide of the type is available in various methods of, for example, chemical synthesis, fermentation or hydrolysis of natural protein, or may also be natural peptides. Any of these are usable herein. One example of natural proteins is soybean protein. This may be hydrolyzed into peptide either chemically or enzymatically, and may be purified through ion-exchange resin. Thus obtained, the short-chain peptide containing ornithine residue (s) in the molecule (short-chain peptide ornithine) is favorable for foods, drinks and others as its mass-scale production is possible at low costs. As compared with ornithine itself, the short-chain peptide ornithine is better in point of the taste, stability, absorbability, safety, etc. Therefore, it is suitable for foods, drinks and others to be taken by persons. The ornithine peptides of the type are, when taken as foods, drinks or the like containing any of them, immediately degraded into ornithine (free body) *in vivo* to exhibit the effect of the free ornithine, like those in medicines.

The active ingredient for use in the invention, especially ornithine (free body, especially L-form), ornithine salts, ornithine derivatives, or a composition that contains such ornithine and branched amino acid(s) may be used in the form of medicines, foods, drinks, etc.

In case where they are used as medicines, they may be in various forms of pharmaceutical preparations that will be mentioned hereinunder.

On the other hand, in case where they are taken as foods, drinks and others, they may be directly taken as they are with no additive thereto, but for more easy taking of them, any of seasonings, flavors and others may be added to them.

The form of the medicines will be mentioned hereinunder. Regarding the form of the foods, drinks and others, they may be in any form of ordinary supplements (nutrient preparations) such as powder, granules, fine granules, tablets, capsules, liquid, jelly, etc. The above-mentioned active ingredient, especially ornithine, ornithine salt(s), ornithine derivative(s), or composition(s) that contain such ornithine or the like along with branched amino acid(s) may be added to already-existing foods, drinks and others to be taken by persons. For example, they may be added to (or incorporated in) drinks, refreshing drinks (soft drinks), yogurt, caramel (candies), jelly, lactic acid bacteria drinks and others to be taken by persons.

In case where the invention is utilized for treating and/or preventing some intended disease and/or for preventing the progress of the disease and/or for ameliorating it, and/or the like the above-mentioned active ingredient for use in the invention, ornithine and/or branched amino acid(s), especially ornithine, ornithine salt(s), ornithine derivative(s) (these are widely within the scope of "ornithine" in the invention) and/or a composition that comprises such ornithine and/or branched amino acid(s), may be administered or given to the diseased persons directly as they are in the form of medicines, foods, drinks and/or the like that contain any of them. However, for further increasing the effect of the active ingredient, any other component that will additively or synergistically act along with the above-mentioned active ingredient may be added to the active ingredient. For example, an antioxidant such as ascorbic acid, cysteine, vitamin E, etc. that may additively or synergistically act along with the effect of the above-mentioned composition may be added to foods, drinks and others that contain the composition, and the combined composition may be administered or given to the diseased persons. This is one preferred administration or ingestion embodiment in the invention.

In case where the remedies/preventives for inflammatory diseases of the invention are administered or given in the form of medicines, foods, drinks and/or the like, the dose (oral administration) or intake thereof shall be controlled, depending on the diseases to which the invention is directed, the condition of the diseases, and the body weight, the age, the constitution, the condition of the patients and the like. In general, however, when ornithine (any of free body, salt(s) thereof, derivative(s) thereof, etc.) is used as the active ingredient, its dose is preferably selected within a range of approximately from 0.25 to 10 g/day, more preferably approximately from 1 to 10 g/day in terms of the dose of (free) ornithine. On the other hand, when branched amino acids are used as the active ingredient, the dose thereof is preferably selected within a range of approximately from 0.25 to 10 g, more preferably approximately from 1 to 10 g in terms of the dose of (free) isoleucine.

In case where the above-mentioned ornithine and branched amino acid(s) are used as combined, the amount thereof shall be suitably selected with reference to the above-mentioned ornithine dose.

In the combined use, the blend ratio of ornithine and branched amino acid(s) is not specifically defined. However, the ratio by weight is preferably such that the branched amino acid(s) account for approximately from 0.1 to 10 relative to ornithine of 1, more preferably approximately from 1 to 5, even more preferably approximately from 1 to 2.

For medicines for parenteral administration, the dose thereof may be selected within a range of approximately from 1/2 to 1/20 the oral dose mentioned above, though depending on the condition of the patients and on the form of the preparations of the medicines, and thee like.

The frequency a day of administration of the medicines or drinking or taking the foods, drinks or others may be once at a time or a few times a day as divided, depending on the condition of the diseases and on the form of the medicines, the foods, the drinks and/or others.

Next described are some embodiments of use as medicines and formulation of medicines.

The medicines of the invention may contain any other various pharmaceutically-acceptable substances (as auxiliary agents, etc.) for preparations, in addition to the active ingredient (one or more) in the invention. The additive agents for preparations may be suitably selected depending on the form of the medicines to be formulated. For example, they include vehicle, diluent, additive, disintegrator, binder, coating agent, wetting agent, gliding agent, lubricant, seasoning, sweetener, solubilizer, etc. Concrete examples of such substances for formulating preparations are magnesium carbonate, titanium dioxide, lactose, mannitol and other saccharides, talc, milk protein, gelatin, starch, cellulose and its derivatives, animal and vegetable oils, polyethylene glycol, as well as solvents such as germ-free water and mono and polyalcohols, for example, glycerol.

The drugs (agents) of the invention may be formulated in various forms of known medicines or medicines that may be developed in future, for example, medicines for oral administration (internal medicines), or other various medicines for other administrations such as intraabdominal administration, percutaneous administration, inhalation administration, eye lotion, etc. For formulating the drugs of the invention into such various forms of medicines, any known method and even any other method that may be developed in future may be employed in any desired manner.

Regarding the form of these various medicines, they may be formulated in any solid or liquid preparations of, for example, granules, powders, coated tablets, tablets, (micro)capsules, suppositories, syrups, juices, suspensions, emulsions, transfusions, solutions for injection, and slow-release preparations of so designed that they may gradually release the active ingredient from them, and the like.

Naturally, the drugs of the invention that are in the form of the preparations exemplified hereinabove shall contain an effective amount of the above-mentioned active ingredient necessary for exhibiting its pharmaceutical potency.

The dose of the drugs (agents) of the invention is as mentioned hereinabove.

Apart from the indispensable active ingredient in the invention, the drugs may contain any other various ingredients. In such cases, the necessary preparations may be formulated on the basis of the pharmaceutical preparation technology known for the additional ingredients or according to the forms of the preparations to be formulated.

As so mentioned hereinabove, another embodiment of the invention resides in a method of treating and/or preventing inflammatory diseases, which comprises giving or administering ornithine and/or branched amino acid(s) to bodies; and still another embodiment thereof resides in a use of ornithine and/or branched amino acid(s) (active ingredient) for remedies/preventives (remedies and/or preventives) for inflammatory diseases, or productions thereof, preferably for remedies/preventives for arthritic diseases, or productions thereof.

These embodiments of the invention are also easy to carry out on the basis of the description relating to the remedies/preventives for inflammatory diseases of the invention described hereinabove and the Examples to be given hereinunder therefor, or, if desired, with reference to the related technology known in the art.

### PREFERRED EMBODIMENTS OF THE INVENTION

The present invention is described further concretely with reference to the following Examples, to which, however, the invention should not be limited.

### (Example 1)

### <Effect of administration of ornithine on murine collagen-induced arthritis>

In an ordinary manner, collagen-induced arthritis (CIA) was induced in DBA-1 mice, on which the pharmaceutical potency of L-ornithine (free body) was investigated. CIA induction in mice was attained in a well-known method, in which mice were sensitized with type-II collagen to make them have experimental arthritis (see Courtenay et al., *Nature,* 283, 666-668, 1980).

Concretely, an emulsion having an antigen amount of 100 µg/0.1 ml/mouse was intracutaneously injected into the tail root of each mouse. Three weeks after the first sensitization, the same emulsion also having an antigen amount of 100 µg/0.1 ml/mouse was again intracutaneously injected into the tail root of each mouse for boosting (additional) immunization, in the same manner as that for the first sensitization. About 4 days after the additional immunization, the mice came to have arthritis, in which reddish edema was seen. The arthritis condition of the mice showed the peak in about 2 weeks after the additional immunization. The level of the edema in the mice was grouped into 4 ranks, and every limb (in four limbs) of each mouse was visually checked. Ornithine (0.03 %) was orally administered to the mice simultaneously with intracutaneous administration of type-II collagen and FCA emulsion. As a result, in the control group, one of the five mice tested showed edema in the leg joints on day 6 after the second subcutaneous administration, two of the five showed it on day 7, and four of the five showed clinical arthritis (joint edema) in 10 days.

On the other hand, in the ornithine-administered group, none of all the tested mice showed edema up to day 13 after the second subcutaneous administration (see Table 1).

The average of the arthritis (joint) scores increased up to 2.8 on day 12 after the second subcutaneous administration in the control group, and the level was kept as such up to day 17. In the ornithine-administered group, no clinical arthritis (joint edema) was shown, and the arthritis (joint) score was zero (see Table 1).

From the above results, it is understood that the ornithine administration significantly retards CIA.

**[Table 1]**

| <Effect of Administration of Ornithine on CIA> | | | | |
|---|---|---|---|---|
| Days after antigenation | Ratio of Individuals with Arthritis (%) | | Arthritis Score | |
| | control group | Ornithine group | Control group | ornithine group |
| 0 | 0 | 0 | 0 | 0 |
| 4 | 0 | 0 | 0 | 0 |
| 6 | 20 | 0 | 0.2 | 0 |
| 8 | 60 | 0 | 0.8 | 0 |
| 10 | 80 | 0 | 1.6 | 0 |
| 12 | 100 | 0 | 3.0 | 0 |
| 14 | 100 | 0 | 3.2 | 0 |

Antigenation means boosting (additional) immunization.

The oral intake of ornithine, calculated from the amount of water taken by the mice, was about 40 mg/kg/day, and this corresponds to a human oral intake of about 2 to 3 g/day.

### (Example 2)

### <Effect of administration of branched amino acid on murine collagen-induced arthritis>

In an ordinary manner, collagen-induced arthritis (CIA) was induced in DBA-1 mice, on which the pharmaceutical potency of a mixture of branched amino acids was investigated in the same manner as in Example 1. The test condition in this is the same as in the above.

A mixture of branched amino acids (L-isoleucine:L-leucine:L-valine = 1:2:1.2 by weight, all free bodies - hereinafter referred to as "BCAA") was orally administered to the mice. The dose was 250 mg/liter. As a result, one of the five mice tested showed edema in the leg joints on day 7 after the second subcutaneous administration, two of the five showed it on day 12, but after that, the number of the affected mice did not increase up to day 18 (see Table 2). The average of the arthritis (joint) scores increased up to 2.8 on day 12 after the second subcutaneous administration in the control group, and the level was kept as such up to day 14. In the BCAA-administered group, it increased up to 1.4 on day 12 after the second subcutaneous administration, but after that, no increase was found (see Table 2).

**[Table 2]**

| <Effect of Administration of BCAA on CIA> | | | | |
|---|---|---|---|---|
| Days after antigenation | Ratio of Individuals with Arthritis (%) | | Arthritis Score | |
| | control group | BCAA group | Control group | BCAA group |
| 0 | 0 | 0 | 0 | 0 |
| 4 | 0 | 0 | 0 | 0 |
| 6 | 20 | 0 | 0.2 | 0 |
| 8 | 60 | 20 | 0.8 | 0.2 |
| 10 | 80 | 20 | 1.6 | 0.6 |
| 12 | 100 | 40 | 3.0 | 1.2 |
| 14 | 100 | 40 | 3.2 | 1.2 |
| 18 | 100 | 40 | 2.2 | 0.8 |

Antigenation means boosting (additional) immunization.

The oral intake of BCAA, calculated from the amount of water taken by the mice, was about 40 mg/kg/day in total, and this corresponds to a human oral intake of about 0.5 to 2 g in terms of each amino acid that constitutes BCAA and about 2 to 3 g/day in total.

### (Example 3)

### <Effect of administration of ornithine on model mice with spontaneously-developed chronic rheumatism>

The pharmaceutical potency of L-ornithine (free body) was investigated on SKG mice, model mice with spontaneously-developed chronic rheumatism (rheumatoid arthritis). In the same manner as in Example 1, 0.03 % L-ornithine (free body) was orally administered to 4-weeks-old or 8-weeks-oldmale SKG mice. As a result, in the groups of the 4-weeks-old mice to which the ornithine administration was started, the mice having turned 10 weeks old developed the disease symptom in both the control group and the ornithine-administered group. However, the score average of the mice having turned 11 weeks old in the control group was 0.74, while that in the ornithine-administered group was 0.18, or that is, depressed. Further, in the control group, the arthritis (joint) score average of the mice having turned 20 weeks old was 3.1, while that in the ornithine-administered group was 2.0, or that is, the score reduction in the ornithine administered group was 35 % as compared with the control group. In the group of the mice to which ornithine was administered when they were 4 weeks old, the disease development was depressed from the first. In the group of the mice to which ornithine was administered when they were 8 weeks old, the disease development was depressed in 2 weeks, but the effect of the ornithine administration to the mice of this group was lower than that to the 4-weeks-old mice of the other group.

The above-mentioned results support the pharmaceutical potency of ornithine to significantly retard the clinical arthritis (joint edema) in the model SKG mice with spontaneously-developed chronic rheumatism (articular rheumatism). Regarding the administration time, the early-stage administration of ornithine was more effective (see Fig. 1).

### (Example 4)

### <Effect of administration of branched amino acid on model mice with spontaneously-developed chronic rheumatism (articular rheumatism)>

The pharmaceutical potency of the above-mentioned BCAA, mixture of branched amino acids, was investigated on SKG mice, model mice with spontaneously-developed chronic rheumatism (articular rheumatism). In the same manner as in Example 2, BCAA was orally administered to 4-weeks-old (before RA development) or 12-weeks-old (after RA development), male SKG mice. The dose to each mouse was 250 mg/liter. In the groups of the 4-weeks-old mice to which the BCAA administration was started, the mice having turned 10 weeks old developed the disease symptom in both the control group and the BCAA-administered group.

As a result of the above, the clinical arthritis score of the mice having turned 11 weeks old was 0.5 in the control group, but was 0.24 in the BCAA-administered group. This indicates a significant depression (50 %) in the disease development in the BCAA-administered group. In addition, the score of the mice having turned 15 weeks old in the control group was 1.94, while that in the BCAA-administered group was 0.5, and this also indicates a significant depression in the disease development in the BCAA-administered group (see Fig. 2).

### (Example 5)

### <Dose-dependent (response) effect of administration of ornithine on murine collagen-induced arthritis>

Collagen-induced arthritis (CIA) was induced in mice in the same manner as above, and the mice was tested also in the same manner as above. Three different types of aqueous solutions of 0.15%, 0.03% or 0.006% (all by weight) L-ornithine (free body) were prepared, and any one of these was administered to each mouse. As a result, the L-ornithine administration showed a dose-response (dependent) effect of retarding clinical arthritis. The mice were judged on day 14 after the boosting (additional) immunization. The results are given in Table 3.

The amount of the aqueous solution taken by the mice was almost the same in all the test groups.

**[Table 3]**

| <Dose-response Effect of Ornithine Administration on CIA> | | |
|---|---|---|
| L-ornithine (wt.%) | Ratio of Individuals with Arthritis (%) | Arthritis Score |
| Control Group | 100 | 3.3 |
| 0.006 % | 40 | 1.0 |
| 0.03 % | 20 | 0.6 |
| 0.15 % | 10 | 0.2 |

### (Example 6)

### <Dose-response (dependent) effect of ornithine administration to SKG mice>

Similarly, SKG mice, model mice with spontaneously-developed chronic rheumatism was tested for the dose-dependent effect of ornithine administration thereto.

Three different types of aqueous solutions of 0.15 %, 0.03 % or 0.006 % (all by weight) L-ornithine (free body) were prepared and tested in the same manner as above. As a result, the L-ornithine administration showed a dose-dependent (response) effect of retarding clinical arthritis. The results are shown in Fig. 3.

The amount of the aqueous solution taken by the mice was almost the same in all the test groups.

### (Example 7)

### <Effect of ornithine administration to rats with adjuvant-induced arthritis>

In an ordinary manner, adjuvant-induced arthritis (AA) was induced in Lewis rats, on which the pharmaceutical potency of L-ornithine (free body) was investigated. AA induction in rats was attained in a well-known laboratory method (see Taurog et al., *Meth. Enzymolo.,* 162, 339-355, 1988). Concretely, 50 µl/rat of 1 % (w/v) adjuvant was administered to the bottom of the right foot of each rat. One, two and three weeks after the administration, the volume of the edema in the bottom of the right foot of each rat was measured by the use of a device for measuring rats' hind leg (foot) edema. Based on this as a ratio to the volume in the control group, the ornithine administration effect was evaluated. Ornithine (0.03 %) was orally administered simultaneously with the adjuvant administration. Based on the reference value, 100 % of the rats in the control group with physiological saline administration, the edema of the ornithine-administered group mice was 25 % (on one week), and this supports the edema suppression in the ornithine-administered group. The results are given in Table 4.

The above-mentioned results confirm that ornithine significantly retards clinical arthritis not only in CIA models and SKG models but also in AA models.

**[Table 4]**

| <Effect of Administration of Ornithine on AA Rats> (clinical arthritis (%) - ratio to control group) | | | |
|---|---|---|---|
| Drug | Days after Adjuvant Administration | | |
| | 7 | 14 | 21 |
| Physiological Saline | 100 | 100 | 100 |
| Ornithine | 25 | 20 | 30 |

The dose is the same as in Example 1. 0.03 % ornithine was orally administered.

On day 7, 14 and 21 after the adjuvant administration, the rats were checked for edema.

### (Example 8)

### <Effect of ornithine administration to mice with collagen-induced arthritis as solid feed>

In an ordinary manner, collagen-induced arthritis (CIA) was induced in DBA-1 mice, on which the pharmaceutical potency of L-ornithine (free body) contained in solid feed was investigated in the same manner as in Example 1. The test condition was the same as above. As a result, in the control group, four of the five mice developed clinical arthritis in 14 days after the second subcutaneous administration; but in the ornithine-administered group, no mice developed it within 14 days after the second subcutaneous administration. The data of the edema development ratio (%) are given in Table 5.

The above-mentioned results confirm that ornithine is effective for retarding clinical arthritis in mice not only in oral administration with free drink of water but also in feeding as solid feed.

**[Table 5]**

| Edema Development Ratio (%) | | | |
|---|---|---|---|
| Drug | Days after Boosting Immunization | | |
| | 7 | 10 | 14 |
| Solid Feed Alone | 40 | 80 | 100 |
| L-Ornithine-Containing Solid Feed | 0 | 20 | 20 |

The L-ornithine-containing solid feed was so controlled that 100 g of the feed contains 30 mg of L-ornithine (free body) .

The oral ornithine intake, calculated from the amount of the feed taken by the mice, was about 46 mg/kg/day, and this corresponds to a human oral intake of about 2 to 3 g/day.

### (Example 9)

### <Effect of combination of ornithine and branched amino acids to mice with collagen-induced arthritis>

Collagen-induced arthritis (CIA) was induced in DBA-1 mice in an ordinary manner, and both L-ornithine (free body) (0.01 %) and the above-mentioned, specific-ratio mixture BCAA of branched chain amino acids (125 mg/liter) were orally administered to them with free drink of water. In the control group, ten of the ten mice tested showed edema in their leg joints on day 12 after the second subcutaneous administration; in the ornithine or BCAA single administration group, three of the ten mice tested showed edema; and in the ornithine and BCAA combined administration group, no mice showed edema. The arthritis (joint) score average was 0.8 in the ornithine single administration group, and 1.2 in the BCAA single administration group as compared with 3.2 on day 12 after the second subcutaneous administration in the control group. However, it was 0 in the combined administration group. The results are given in Table 6.

**[Table 6]**

| <Effect of Combined Ornithine and BCAA on CIA> | | |
|---|---|---|
| Drug | Ratio of Individuals with Arthritis (%) | Arthritis Score |
| Control Group | 100 | 3.2 |
| Ornithine | 30 | 0.8 |
| BCAA | 30 | 1.2 |
| Combined Group | 0 | 0 |

The oral intake of ornithine, calculated from the amount of water taken by the mice, was about 15 mg/kg/day, and this corresponds to a human oral intake of about 2 to 3 g/day. The oral intake of BCAA was about 20 mg/kg/day in total, and this corresponds to a human oral intake of about 0.25 to 2 g in terms of each amino acid that constitutes BCAA and about 1 to 1.5 g/day in total.

### ADVANTAGES OF THE INVENTION

The remedy(ies)/preventive(s) for inflammatory disease(s) of the invention that contain ornithine and/or branched amino acid(s) as the active ingredient are extremely effective for treating and/or preventing inflammatory disease(s) such as typically chronic rheumatism (articular rheumatism), for preventing the progress of the disease(s), and/or for ameliorating the disease (s), and/or the like. Since they have no or few side effects in treating and/or preventing such inflammatory disease(s), preventing the progress of the disease(s) and/or ameliorating the disease(s), they may be in any form of not only medicines (including transfusions, supplements (nutrient preparations), etc.) but also foods and/or drinks (including medical foods, health foods, specific (specified) health foods, etc.) in expectation of preventing and/or ameliorating the disease(s).

When ornithine is combined with branched amino acid(s), especially with a mixture of leucine, isoleucine and valine, the above-mentioned effect is further increased.

According to the invention, there are further provided a method of using the drug(s) (agent(s)) for treating or preventing inflammatory disease (s), and also a use of the active ingredient for remedy(ies)/preventive(s) for inflammatory diseases) or production(s) thereof.

Accordingly, the invention is extremely useful in the field of industry, especially in various fields of medical treatments, medicines, foods, etc.

## Claims

1. A remedy/preventive for inflammatory disease, containing ornithine and/or branched amino acid(s) as an active ingredient, in which the ornithine and branched amino acid(s) each may be in any form of free amino acid(s), salt(s) thereof and derivative(s) thereof that may be converted into the free amino acid(s) *in vivo,* if any.

2. The remedy/preventive for inflammatory disease as claimed in claim 1, wherein the ornithine and/or branched amino acid(s) are in the L-form.

3. The remedy/preventive for inflammatory disease as claimed in claim 1, wherein the branched amino acid(s) are at least one of leucine, isoleucine and valine.

4. The remedy/preventive for inflammatory disease as claimed in any of claims 1 to 3, which contains ornithine and branched amino acid(s).

5. The remedy/preventive for inflammatory disease as claimed in any of claims 1 to 4, which is for preventing and treating an arthritic disease.

6. The remedy/preventive for inflammatory disease as claimed in any of claims 1 to 5, wherein the ornithine is an ornithine derivative and the ornithine derivative is a peptide that contains ornithine as the constitutive amino acid thereof.

7. The remedy/preventive for inflammatory disease as claimed in any of claims 1 to 6, which is suitable to oral administration or oral taking.

8. The remedy/preventive for inflammatory disease as claimed in any of claims 1 to 7, which is in the form of medicine, or food or drink.

9. The remedy/preventive for inflammatory disease as claimed in claim 8, wherein the medicine is in any form of supplement or transfusion, and the food or drink is in any form of medical food, health food and specified health food.

10. A method for treating or preventing an inflammatory disease, which comprises giving or administering ornithine and/or branched amino acid(s) to a living body and in which the ornithine and branched amino acid(s) each may be in any form of free amino acid(s), salt(s) thereof and derivative(s) thereof that may be converted into the free amino acid(s) *in vivo,* if any.

11. The method as claimed in claim 10, wherein the form to be taken or administered is in the form of the remedy/preventive for inflammatory disease described in any of claims 1 to 7.

12. The method as claimed in claim 10 or 11, wherein the form to be taken or administered is in a form of medicine, or food or drink.

13. The method as claimed in any of claims 10 to 12, which is a method for preventing or treating an arthritic disease.

14. A use of ornithine and/or branched amino acid (s) for remedy/preventive for inflammatory disease, in which the ornithine and branched amino acid(s) each may be in any form of free amino acid(s), salt(s) thereof and derivative(s) thereof that may be converted into the free amino acid(s) *in vivo,* if any.

15. The use as claimed in claim 14, which is in a form wherein the remedy/preventive for inflammatory disease is used in a medicine, or a food or drink.

16. The use as claimed in claim 14, wherein the remedy/preventive for inflammatory disease is one described in any of claims 1 to 7.

17. Use as claimed in any of claims 14 to 16, wherein the remedy/preventive for inflammatory disease is a remedy and/or a preventive for arthritic disease.
